# EUROPEAN PATENT APPLICATION

(11) **EP 1 738 763 A1**
(43) Date of publication of application: **03.01.2007**
(21) Application number: 05254123.2
(22) Date of filing: 30.06.2005
(51) Int. Cl.: A61K 38/17, A61P 25/00

(54) **Use of complement inhibitory proteins to treat spinal cord injury**

(71) Applicant: AVANT Immunotherapeutics, Inc., Needham, Massachusetts 02139 (US)
(72) Inventor: Li, Liang-man,, China Medical Univ. 86-024-23256666-6231 (CN); Zhu, Yue, China Medical Univ. 86-024-23256666-6231 (CN); Fan, Guang-yu, China Medical Univ. 86-024-23256666-6231 (CN)
(74) Representative: Silveston, Judith

(57) **Abstract**

Trauma to the spinal cord initiates an inflammatory response that results in secondary injury to the surrounding tissue, thereby exacerbating the effects of the initial injury. These secondary injury effects are contributed to, in part, by the activation of complement and the associated inflammatory reaction at the site of injury. The present invention describes a method for treating and/or ameliorating these secondary effects and improving locomotor function of a vertebrate that has suffered a spinal cord injury by administering a complement inhibitory protein to the individual as soon as possible after the initial injury occurs. According to this method, by inhibiting the activation of complement, inflammation and the resulting secondary tissue injury are reduced, thereby improving the prognosis of a patient suffering a spinal cord injury.

SEQUENCE LISTING

<110> AVANT Immunotherapeutics, Inc.
LI, Liang-man
FAN, Guang-yu
ZHU, Yu

<120> Use of Complement Inhibitory Proteins to Treat Spinal Cord Injury

<130> AVA-445.0 US

<140> (To Be Assigned)
<141> 2005-06-27

<160> 3

<170> PatentIn version 3.3

<210> 1
<211> 6117
<212> DNA
<213> Homo sapiens

<400> 1
atgggggcct cttctccaag aagcccggag cctgtcgggc cgccggcgcc cggtctcccc 60

ttctgctgcg gaggatccct gctggcggtt gtggtgctgc ttgcgctgcc ggtggcctgg 120

ggtcaatgca atgccccaga atggcttcca tttgccaggc ctaccaacct aactgatgag 180

tttgagtttc ccattgggac atatctgaac tatgaatgcc gccctggtta ttccggaaga 240

ccgttttcta tcatctgcct aaaaaactca gtctggactg gtgctaagga caggtgcaga 300

cgtaaatcat gtcgtaatcc tccagatcct gtgaatggca tggtgcatgt gatcaaaggc 360

atccagttcg gatcccaaat taaatattct tgtactaaag gataccgact cattggttcc 420

tcgtctgcca catgcatcat ctcaggtgat actgtcattt gggataatga aacacctatt 480

tgtgacagaa ttccttgtgg gctacccccc accatcacca atggagattt cattagcacc 540

aacagagaga attttcacta tggatcagtg gtgacctacc gctgcaatcc tggaagcgga 600

gggagaaagg tgtttgagct tgtgggtgag ccctccatat actgcaccag caatgacgat 660

caagtgggca tctggagcgg ccccgcccct cagtgcatta tacctaacaa atgcacgcct 720

ccaaatgtgg aaaatggaat attggtatct gacaacagaa gcttattttc cttaaatgaa 780

gttgtggagt ttaggtgtca gcctggcttt gtcatgaaag gaccccgccg tgtgaagtgc 840

caggccctga acaaatggga gccggagcta ccaagctgct ccagggtatg tcagccacct 900

ccagatgtcc tgcatgctga gcgtacccaa agggacaagg acaacttttc acctgggcag 960

gaagtgttct acagctgtga gcccggctac gacctcagag gggctgcgtc tatgcgctgc 1020

acaccccagg gagactggag ccctgcagcc cccacatgtg aagtgaaatc ctgtgatgac 1080

ttcatgggcc aacttcttaa tggccgtgtg ctatttccag taaatctcca gcttggagca 1140

aaagtggatt ttgtttgtga tgaaggattt caattaaaag gcagctctgc tagttactgt 1200

gtcttggctg gaatggaaag cctttggaat agcagtgttc cagtgtgtga acaaatcttt 1260
tgtccaagtc ctccagttat tcctaatggg agacacacag gaaaacctct ggaagtcttt 1320
ccctttggaa aagcagtaaa ttacacatgc gacccccacc cagacagagg gacgagcttc 1380
gacctcattg gagagagcac catccgctgc acaagtgacc ctcaagggaa tggggtttgg 1440
agcagccctg cccctcgctg tggaattctg ggtcactgtc aagccccaga tcattttctg 1500
tttgccaagt tgaaaaccca aaccaatgca tctgactttc ccattgggac atctttaaag 1560
tacgaatgcc gtcctgagta ctacgggagg ccattctcta tcacatgtct agataacctg 1620
gtctggtcaa gtcccaaaga tgtctgtaaa cgtaaatcat gtaaaactcc tccagatcca 1680
gtgaatggca tggtgcatgt gatcacagac atccaggttg gatccagaat caactattct 1740
tgtactacag ggcaccgact cattggtcac tcatctgctg aatgtatcct ctcgggcaat 1800
gctgcccatt ggagcacgaa gccgccaatt tgtcaacgaa ttccttgtgg gctacccccc 1860
accatcgcca atggagattt cattagcacc aacagagaga attttcacta tggatcagtg 1920
gtgacctacc gctgcaatcc tggaagcgga gggagaaagg tgtttgagct tgtgggtgag 1980
ccctccatat actgcaccag caatgacgat caagtgggca tctggagcgg cccggcccct 2040
cagtgcatta tacctaacaa atgcacgcct ccaaatgtgg aaaatggaat attggtatct 2100
gacaacagaa gcttattttc cttaaatgaa gttgtggagt ttaggtgtca gcctggcttt 2160
gtcatgaaag gaccccgccg tgtgaagtgc caggccctga acaaatggga gccggagcta 2220
ccaagctgct ccagggtatg tcagccacct ccagatgtcc tgcatgctga gcgtacccaa 2280
agggacaagg acaacttttc acccgggcag gaagtgttct acagctgtga gcccggctat 2340
gacctcagag gggctgcgtc tatgcgctgc acaccccagg gagactggag ccctgcagcc 2400
cccacatgtg aagtgaaatc ctgtgatgac ttcatgggcc aacttcttaa tggccgtgtg 2460
ctatttccag taaatctcca gcttggagca aaagtggatt ttgtttgtga tgaaggattt 2520
caattaaaag gcagctctgc tagttattgt gtcttggctg gaatggaaag cctttggaat 2580
agcagtgttc cagtgtgtga acaaatcttt tgtccaagtc ctccagttat tcctaatggg 2640
agacacacag gaaaacctct ggaagtcttt ccctttggaa aagcagtaaa ttacacatgc 2700
gacccccacc cagacagagg gacgagcttc gacctcattg gagagagcac catccgctgc 2760
acaagtgacc ctcaagggaa tggggtttgg agcagccctg cccctcgctg tggaattctg 2820
ggtcactgtc aagccccaga tcattttctg tttgccaagt tgaaaaccca aaccaatgca 2880
tctgactttc ccattgggac atctttaaag tacgaatgcc gtcctgagta ctacgggagg 2940
ccattctcta tcacatgtct agataacctg gtctggtcaa gtcccaaaga tgtctgtaaa 3000
cgtaaatcat gtaaaactcc tccagatcca gtgaatggca tggtgcatgt gatcacagac 3060

atccaggttg gatccagaat caactattct tgtactacag ggcaccgact cattggtcac 3120
tcatctgctg aatgtatcct ctcaggcaat actgcccatt ggagcacgaa gccgccaatt 3180
tgtcaacgaa ttccttgtgg gctaccccca accatcgcca atggagattt cattagcacc 3240
aacagagaga attttcacta tggatcagtg gtgacctacc gctgcaatct tggaagcaga 3300
gggagaaagg tgtttgagct tgtgggtgag ccctccatat actgcaccag caatgacgat 3360
caagtgggca tctggagcgg ccccgcccct cagtgcatta tacctaacaa atgcacgcct 3420
ccaaatgtgg aaaatggaat attggtatct gacaacagaa gcttattttc cttaaatgaa 3480
gttgtggagt ttaggtgtca gcctggcttt gtcatgaaag gaccccgccg tgtgaagtgc 3540
caggccctga acaaatggga gccagagtta ccaagctgct ccagggtgtg tcagccgcct 3600
ccagaaatcc tgcatggtga gcatacccca agccatcagg acaacttttc acctgggcag 3660
gaagtgttct acagctgtga gcctggctat gacctcagag gggctgcgtc tctgcactgc 3720
acaccccagg gagactggag ccctgaagcc ccgagatgtg cagtgaaatc ctgtgatgac 3780
ttcttgggtc aactccctca tggccgtgtg ctatttccac ttaatctcca gcttggggca 3840
aaggtgtcct ttgtctgtga tgaagggttt cgcttaaagg gcagttccgt tagtcattgt 3900
gtcttggttg gaatgagaag cctttggaat aacagtgttc ctgtgtgtga acatatcttt 3960
tgtccaaatc ctccagctat ccttaatggg agacacacag gaactccctc tggagatatt 4020
ccctatggaa aagaaatatc ttacacatgt gacccccacc cagacagagg gatgaccttc 4080
aacctcattg gggagagcac catccgctgc acaagtgacc ctcatgggaa tggggtttgg 4140
agcagccctg cccctcgctg tgaactttct gttcgtgctg gtcactgtaa aaccccagag 4200
cagtttccat ttgccagtcc tacgatccca attaatgact ttgagtttcc agtcgggaca 4260
tctttgaatt atgaatgccg tcctgggtat tttgggaaaa tgttctctat ctcctgccta 4320
gaaaacttgg tctggtcaag tgttgaagac aactgtagac gaaaatcatg tggacctcca 4380
ccagaaccct tcaatggaat ggtgcatata aacacagata cacagtttgg atcaacagtt 4440
aattattctt gtaatgaagg gtttcgactc attggttccc catctactac ttgtctcgtc 4500
tcaggcaata atgtcacatg ggataagaag gcacctattt gtgagatcat atcttgtgag 4560
ccacctccaa ccatatccaa tggagacttc tacagcaaca atagaacatc ttttcacaat 4620
ggaacggtgg taacttacca gtgccacact ggaccagatg gagaacagct gtttgagctt 4680
gtgggagaac ggtcaatata ttgcaccagc aaagatgatc aagttggtgt ttggagcagc 4740
cctccccctc ggtgtatttc tactaataaa tgcacagctc cagaagttga aaatgcaatt 4800
agagtaccag gaaacaggag tttcttttcc ctcactgaga tcatcagatt tagatgtcag 4860
cccgggtttg tcatggtagg gtcccacact gtgcagtgcc agaccaatgg cagatggggg 4920
cccaagctgc cacactgctc cagggtgtgt cagccgcctc cagaaatcct gcatggtgag 4980

cataccctaa gccatcagga caacttttca cctgggcagg aagtgttcta cagctgtgag 5040

cccagctatg acctcagagg ggctgcgtct ctgcactgca cgccccaggg agactggagc 5100

cctgaagccc ctagatgtac agtgaaatcc tgtgatgact tcctgggcca actccctcat 5160

ggccgtgtgc tacttccact taatctccag cttggggcaa aggtgtcctt tgtttgcgat 5220

gaagggttcc gattaaaagg caggtctgct agtcattgtg tcttggctgg aatgaaagcc 5280

ctttggaata gcagtgttcc agtgtgtgaa caaatctttt gtccaaatcc tccagctatc 5340

cttaatggga gacacacagg aactcccttt ggagatattc cctatggaaa agaaatatct 5400

tacgcatgcg acacccaccc agacagaggg atgaccttca acctcattgg ggagagctcc 5460

atccgctgca caagtgaccc tcaagggaat ggggtttgga gcagccctgc ccctcgctgt 5520

gaactttctg ttcctgctgc ctgcccacat ccacccaaga tccaaaacgg gcattacatt 5580

ggaggacacg tatctctata tcttcctggg atgacaatca gctacacttg tgaccccggc 5640

tacctgttag tgggaaaggg cttcattttc tgtacagacc agggaatctg gagccaattg 5700

gatcattatt gcaaagaagt aaattgtagc ttcccactgt ttatgaatgg aatctcgaag 5760

gagttagaaa tgaaaaaagt atatcactat ggagattatg tgactttgaa gtgtgaagat 5820

gggtatactc tggaaggcag tccctggagc cagtgccagg cggatgacag atgggaccct 5880

cctctggcca aatgtacctc tcgtgcacat gatgctctca tagttggcac tttatctggt 5940

acgatcttct ttattttact catcattttc ctctcttgga taattctaaa gcacagaaaa 6000

ggcaataatg cacatgaaaa ccctaaagaa gtggctatcc atttacattc tcaaggaggc 6060

agcagcgttc atccccgaac tctgcaaaca aatgaagaaa atagcagggt ccttcct 6117

<210> 2
<211> 1998
<212> PRT
<213> Homo sapiens

<220>
<221> MISC_FEATURE
<222> (1)..(1998)
<223> Full Length Protein

<400> 2

Gln Cys Asn Ala Pro Glu Trp Leu Pro Phe Ala Arg Pro Thr Asn Leu
1               5                   10                  15

Thr Asp Glu Phe Glu Phe Pro Ile Gly Thr Tyr Leu Asn Tyr Glu Cys
            20                  25                  30

Arg Pro Gly Tyr Ser Gly Arg Pro Phe Ser Ile Ile Cys Leu Lys Asn
        35                  40                  45

Ser Val Trp Thr Gly Ala Lys Asp Arg Cys Arg Arg Lys Ser Cys Arg
    50                  55                  60

Asn Pro Pro Asp Pro Val Asn Gly Met Val His Val Ile Lys Gly Ile
65                  70                  75                  80

Gln Phe Gly Ser Gln Ile Lys Tyr Ser Cys Thr Lys Gly Tyr Arg Leu
                85                  90                  95

Ile Gly Ser Ser Ser Ala Thr Cys Ile Ile Ser Gly Asp Thr Val Ile
            100                 105                 110

Trp Asp Asn Glu Thr Pro Ile Cys Asp Arg Ile Pro Cys Gly Leu Pro
        115                 120                 125

Pro Thr Ile Thr Asn Gly Asp Phe Ile Ser Thr Asn Arg Glu Asn Phe
    130                 135                 140

His Tyr Gly Ser Val Val Thr Tyr Arg Cys Asn Pro Gly Ser Gly Gly
145                 150                 155                 160

Arg Lys Val Phe Glu Leu Val Gly Glu Pro Ser Ile Tyr Cys Thr Ser
                165                 170                 175

Asn Asp Asp Gln Val Gly Ile Trp Ser Gly Pro Ala Pro Gln Cys Ile
            180                 185                 190

Ile Pro Asn Lys Cys Thr Pro Pro Asn Val Glu Asn Gly Ile Leu Val
        195                 200                 205

Ser Asp Asn Arg Ser Leu Phe Ser Leu Asn Glu Val Val Glu Phe Arg
    210                 215                 220

Cys Gln Pro Gly Phe Val Met Lys Gly Pro Arg Arg Val Lys Cys Gln
225                 230                 235                 240

Ala Leu Asn Lys Trp Glu Pro Glu Leu Pro Ser Cys Ser Arg Val Cys
                245                 250                 255

Gln Pro Pro Pro Asp Val Leu His Ala Glu Arg Thr Gln Arg Asp Lys
            260                 265                 270

Asp Asn Phe Ser Pro Gly Gln Glu Val Phe Tyr Ser Cys Glu Pro Gly
        275                 280                 285

Tyr Asp Leu Arg Gly Ala Ala Ser Met Arg Cys Thr Pro Gln Gly Asp

290                 295                 300

Trp Ser Pro Ala Ala Pro Thr Cys Glu Val Lys Ser Cys Asp Asp Phe
305                 310                 315                 320

Met Gly Gln Leu Leu Asn Gly Arg Val Leu Phe Pro Val Asn Leu Gln
                325                 330                 335

Leu Gly Ala Lys Val Asp Phe Val Cys Asp Glu Gly Phe Gln Leu Lys
            340                 345                 350

Gly Ser Ser Ala Ser Tyr Cys Val Leu Ala Gly Met Glu Ser Leu Trp
        355                 360                 365

Asn Ser Ser Val Pro Val Cys Glu Gln Ile Phe Cys Pro Ser Pro Pro
    370                 375                 380

Val Ile Pro Asn Gly Arg His Thr Gly Lys Pro Leu Glu Val Phe Pro
385                 390                 395                 400

Phe Gly Lys Ala Val Asn Tyr Thr Cys Asp Pro His Pro Asp Arg Gly
                405                 410                 415

Thr Ser Phe Asp Leu Ile Gly Glu Ser Thr Ile Arg Cys Thr Ser Asp
            420                 425                 430

Pro Gln Gly Asn Gly Val Trp Ser Ser Pro Ala Pro Arg Cys Gly Ile
        435                 440                 445

Leu Gly His Cys Gln Ala Pro Asp His Phe Leu Phe Ala Lys Leu Lys
    450                 455                 460

Thr Gln Thr Asn Ala Ser Asp Phe Pro Ile Gly Thr Ser Leu Lys Tyr
465                 470                 475                 480

Glu Cys Arg Pro Glu Tyr Tyr Gly Arg Pro Phe Ser Ile Thr Cys Leu
                485                 490                 495

Asp Asn Leu Val Trp Ser Ser Pro Lys Asp Val Cys Lys Arg Lys Ser
            500                 505                 510

Cys Lys Thr Pro Pro Asp Pro Val Asn Gly Met Val His Val Ile Thr
        515                 520                 525

Asp Ile Gln Val Gly Ser Arg Ile Asn Tyr Ser Cys Thr Thr Gly His
    530                 535                 540

Arg Leu Ile Gly His Ser Ser Ala Glu Cys Ile Leu Ser Gly Asn Ala
545                 550                 555                 560

Ala His Trp Ser Thr Lys Pro Pro Ile Cys Gln Arg Ile Pro Cys Gly
                565                 570                 575

Leu Pro Pro Thr Ile Ala Asn Gly Asp Phe Ile Ser Thr Asn Arg Glu
            580                 585                 590

Asn Phe His Tyr Gly Ser Val Val Thr Tyr Arg Cys Asn Pro Gly Ser
        595                 600                 605

Gly Gly Arg Lys Val Phe Glu Leu Val Gly Glu Pro Ser Ile Tyr Cys
    610                 615                 620

Thr Ser Asn Asp Asp Gln Val Gly Ile Trp Ser Gly Pro Ala Pro Gln
625                 630                 635                 640

Cys Ile Ile Pro Asn Lys Cys Thr Pro Pro Asn Val Glu Asn Gly Ile
                645                 650                 655

Leu Val Ser Asp Asn Arg Ser Leu Phe Ser Leu Asn Glu Val Val Glu
            660                 665                 670

Phe Arg Cys Gln Pro Gly Phe Val Met Lys Gly Pro Arg Arg Val Lys
        675                 680                 685

Cys Gln Ala Leu Asn Lys Trp Glu Pro Glu Leu Pro Ser Cys Ser Arg
    690                 695                 700

Val Cys Gln Pro Pro Pro Asp Val Leu His Ala Glu Arg Thr Gln Arg
705                 710                 715                 720

Asp Lys Asp Asn Phe Ser Pro Gly Gln Glu Val Phe Tyr Ser Cys Glu
                725                 730                 735

Pro Gly Tyr Asp Leu Arg Gly Ala Ala Ser Met Arg Cys Thr Pro Gln
            740                 745                 750

Gly Asp Trp Ser Pro Ala Ala Pro Thr Cys Glu Val Lys Ser Cys Asp
        755                 760                 765

Asp Phe Met Gly Gln Leu Leu Asn Gly Arg Val Leu Phe Pro Val Asn
    770                 775                 780

Leu Gln Leu Gly Ala Lys Val Asp Phe Val Cys Asp Glu Gly Phe Gln
785                 790                 795                 800

Leu Lys Gly Ser Ser Ala Ser Tyr Cys Val Leu Ala Gly Met Glu Ser
                805                 810                 815

Leu Trp Asn Ser Ser Val Pro Val Cys Glu Gln Ile Phe Cys Pro Ser
            820                 825                 830

Pro Pro Val Ile Pro Asn Gly Arg His Thr Gly Lys Pro Leu Glu Val
        835                 840                 845

Phe Pro Phe Gly Lys Ala Val Asn Tyr Thr Cys Asp Pro His Pro Asp
    850                 855                 860

Arg Gly Thr Ser Phe Asp Leu Ile Gly Glu Ser Thr Ile Arg Cys Thr
865                 870                 875                 880

Ser Asp Pro Gln Gly Asn Gly Val Trp Ser Ser Pro Ala Pro Arg Cys
                885                 890                 895

Gly Ile Leu Gly His Cys Gln Ala Pro Asp His Phe Leu Phe Ala Lys
            900                 905                 910

Leu Lys Thr Gln Thr Asn Ala Ser Asp Phe Pro Ile Gly Thr Ser Leu
        915                 920                 925

Lys Tyr Glu Cys Arg Pro Glu Tyr Tyr Gly Arg Pro Phe Ser Ile Thr
    930                 935                 940

Cys Leu Asp Asn Leu Val Trp Ser Ser Pro Lys Asp Val Cys Lys Arg
945                 950                 955                 960

Lys Ser Cys Lys Thr Pro Pro Asp Pro Val Asn Gly Met Val His Val
                965                 970                 975

Ile Thr Asp Ile Gln Val Gly Ser Arg Ile Asn Tyr Ser Cys Thr Thr
            980                 985                 990

Gly His Arg Leu Ile Gly His Ser  Ser Ala Glu Cys Ile  Leu Ser Gly
        995                 1000                 1005

Asn Thr  Ala His Trp Ser Thr  Lys Pro Pro Ile Cys  Gln Arg Ile
    1010                 1015                 1020

Pro Cys  Gly Leu Pro Pro Thr  Ile Ala Asn Gly Asp  Phe Ile Ser
    1025                 1030                 1035

Thr Asn  Arg Glu Asn Phe His  Tyr Gly Ser Val Val  Thr Tyr Arg
    1040                 1045                 1050

Cys Asn Leu Gly Ser Arg Gly Arg Lys Val Phe Glu Leu Val Gly
    1055                    1060                    1065

Glu Pro Ser Ile Tyr Cys Thr Ser Asn Asp Asp Gln Val Gly Ile
    1070                    1075                    1080

Trp Ser Gly Pro Ala Pro Gln Cys Ile Ile Pro Asn Lys Cys Thr
    1085                    1090                    1095

Pro Pro Asn Val Glu Asn Gly Ile Leu Val Ser Asp Asn Arg Ser
    1100                    1105                    1110

Leu Phe Ser Leu Asn Glu Val Val Glu Phe Arg Cys Gln Pro Gly
    1115                    1120                    1125

Phe Val Met Lys Gly Pro Arg Arg Val Lys Cys Gln Ala Leu Asn
    1130                    1135                    1140

Lys Trp Glu Pro Glu Leu Pro Ser Cys Ser Arg Val Cys Gln Pro
    1145                    1150                    1155

Pro Pro Glu Ile Leu His Gly Glu His Thr Pro Ser His Gln Asp
    1160                    1165                    1170

Asn Phe Ser Pro Gly Gln Glu Val Phe Tyr Ser Cys Glu Pro Gly
    1175                    1180                    1185

Tyr Asp Leu Arg Gly Ala Ala Ser Leu His Cys Thr Pro Gln Gly
    1190                    1195                    1200

Asp Trp Ser Pro Glu Ala Pro Arg Cys Ala Val Lys Ser Cys Asp
    1205                    1210                    1215

Asp Phe Leu Gly Gln Leu Pro His Gly Arg Val Leu Phe Pro Leu
    1220                    1225                    1230

Asn Leu Gln Leu Gly Ala Lys Val Ser Phe Val Cys Asp Glu Gly
    1235                    1240                    1245

Phe Arg Leu Lys Gly Ser Ser Val Ser His Cys Val Leu Val Gly
    1250                    1255                    1260

Met Arg Ser Leu Trp Asn Asn Ser Val Pro Val Cys Glu His Ile
    1265                    1270                    1275

Phe Cys Pro Asn Pro Pro Ala Ile Leu Asn Gly Arg His Thr Gly

1280                    1285                    1290

Thr Pro Ser Gly Asp Ile Pro Tyr Gly Lys Glu Ile Ser Tyr Thr
    1295                    1300                    1305

Cys Asp Pro His Pro Asp Arg Gly Met Thr Phe Asn Leu Ile Gly
    1310                    1315                    1320

Glu Ser Thr Ile Arg Cys Thr Ser Asp Pro His Gly Asn Gly Val
    1325                    1330                    1335

Trp Ser Ser Pro Ala Pro Arg Cys Glu Leu Ser Val Arg Ala Gly
    1340                    1345                    1350

His Cys Lys Thr Pro Glu Gln Phe Pro Phe Ala Ser Pro Thr Ile
    1355                    1360                    1365

Pro Ile Asn Asp Phe Glu Phe Pro Val Gly Thr Ser Leu Asn Tyr
    1370                    1375                    1380

Glu Cys Arg Pro Gly Tyr Phe Gly Lys Met Phe Ser Ile Ser Cys
    1385                    1390                    1395

Leu Glu Asn Leu Val Trp Ser Ser Val Glu Asp Asn Cys Arg Arg
    1400                    1405                    1410

Lys Ser Cys Gly Pro Pro Pro Glu Pro Phe Asn Gly Met Val His
    1415                    1420                    1425

Ile Asn Thr Asp Thr Gln Phe Gly Ser Thr Val Asn Tyr Ser Cys
    1430                    1435                    1440

Asn Glu Gly Phe Arg Leu Ile Gly Ser Pro Ser Thr Thr Cys Leu
    1445                    1450                    1455

Val Ser Gly Asn Asn Val Thr Trp Asp Lys Lys Ala Pro Ile Cys
    1460                    1465                    1470

Glu Ile Ile Ser Cys Glu Pro Pro Pro Thr Ile Ser Asn Gly Asp
    1475                    1480                    1485

Phe Tyr Ser Asn Asn Arg Thr Ser Phe His Asn Gly Thr Val Val
    1490                    1495                    1500

Thr Tyr Gln Cys His Thr Gly Pro Asp Gly Glu Gln Leu Phe Glu
    1505                    1510                    1515

Leu Val Gly Glu Arg Ser Ile Tyr Cys Thr Ser Lys Asp Asp Gln
    1520                1525                1530

Val Gly Val Trp Ser Ser Pro Pro Pro Arg Cys Ile Ser Thr Asn
    1535                1540                1545

Lys Cys Thr Ala Pro Glu Val Glu Asn Ala Ile Arg Val Pro Gly
    1550                1555                1560

Asn Arg Ser Phe Phe Ser Leu Thr Glu Ile Ile Arg Phe Arg Cys
    1565                1570                1575

Gln Pro Gly Phe Val Met Val Gly Ser His Thr Val Gln Cys Gln
    1580                1585                1590

Thr Asn Gly Arg Trp Gly Pro Lys Leu Pro His Cys Ser Arg Val
    1595                1600                1605

Cys Gln Pro Pro Pro Glu Ile Leu His Gly Glu His Thr Leu Ser
    1610                1615                1620

His Gln Asp Asn Phe Ser Pro Gly Gln Glu Val Phe Tyr Ser Cys
    1625                1630                1635

Glu Pro Ser Tyr Asp Leu Arg Gly Ala Ala Ser Leu His Cys Thr
    1640                1645                1650

Pro Gln Gly Asp Trp Ser Pro Glu Ala Pro Arg Cys Thr Val Lys
    1655                1660                1665

Ser Cys Asp Asp Phe Leu Gly Gln Leu Pro His Gly Arg Val Leu
    1670                1675                1680

Leu Pro Leu Asn Leu Gln Leu Gly Ala Lys Val Ser Phe Val Cys
    1685                1690                1695

Asp Glu Gly Phe Arg Leu Lys Gly Arg Ser Ala Ser His Cys Val
    1700                1705                1710

Leu Ala Gly Met Lys Ala Leu Trp Asn Ser Ser Val Pro Val Cys
    1715                1720                1725

Glu Gln Ile Phe Cys Pro Asn Pro Pro Ala Ile Leu Asn Gly Arg
    1730                1735                1740

His Thr Gly Thr Pro Phe Gly Asp Ile Pro Tyr Gly Lys Glu Ile
    1745                1750                1755

Ser Tyr Ala Cys Asp Thr His Pro Asp Arg Gly Met Thr Phe Asn
    1760                1765                1770

Leu Ile Gly Glu Ser Ser Ile Arg Cys Thr Ser Asp Pro Gln Gly
    1775                1780                1785

Asn Gly Val Trp Ser Ser Pro Ala Pro Arg Cys Glu Leu Ser Val
    1790                1795                1800

Pro Ala Ala Cys Pro His Pro Pro Lys Ile Gln Asn Gly His Tyr
    1805                1810                1815

Ile Gly Gly His Val Ser Leu Tyr Leu Pro Gly Met Thr Ile Ser
    1820                1825                1830

Tyr Thr Cys Asp Pro Gly Tyr Leu Leu Val Gly Lys Gly Phe Ile
    1835                1840                1845

Phe Cys Thr Asp Gln Gly Ile Trp Ser Gln Leu Asp His Tyr Cys
    1850                1855                1860

Lys Glu Val Asn Cys Ser Phe Pro Leu Phe Met Asn Gly Ile Ser
    1865                1870                1875

Lys Glu Leu Glu Met Lys Lys Val Tyr His Tyr Gly Asp Tyr Val
    1880                1885                1890

Thr Leu Lys Cys Glu Asp Gly Tyr Thr Leu Glu Gly Ser Pro Trp
    1895                1900                1905

Ser Gln Cys Gln Ala Asp Asp Arg Trp Asp Pro Pro Leu Ala Lys
    1910                1915                1920

Cys Thr Ser Arg Ala His Asp Ala Leu Ile Val Gly Thr Leu Ser
    1925                1930                1935

Gly Thr Ile Phe Phe Ile Leu Leu Ile Ile Phe Leu Ser Trp Ile
    1940                1945                1950

Ile Leu Lys His Arg Lys Gly Asn Asn Ala His Glu Asn Pro Lys
    1955                1960                1965

Glu Val Ala Ile His Leu His Ser Gln Gly Gly Ser Ser Val His
    1970                1975                1980

Pro Arg Thr Leu Gln Thr Asn Glu Glu Asn Ser Arg Val Leu Pro
    1985                1990                1995

<210>  3
<211>  1931
<212>  PRT
<213>  Homo sapiens

<220>
<221>  MISC_FEATURE
<222>  (1)..(1931)
<223>  soluble CR1 polypeptide

<400>  3

Gln Cys Asn Ala Pro Glu Trp Leu Pro Phe Ala Arg Pro Thr Asn Leu
1               5                   10                  15

Thr Asp Glu Phe Glu Phe Pro Ile Gly Thr Tyr Leu Asn Tyr Glu Cys
            20                  25                  30

Arg Pro Gly Tyr Ser Gly Arg Pro Phe Ser Ile Ile Cys Leu Lys Asn
        35                  40                  45

Ser Val Trp Thr Gly Ala Lys Asp Arg Cys Arg Arg Lys Ser Cys Arg
    50                  55                  60

Asn Pro Pro Asp Pro Val Asn Gly Met Val His Val Ile Lys Gly Ile
65                  70                  75                  80

Gln Phe Gly Ser Gln Ile Lys Tyr Ser Cys Thr Lys Gly Tyr Arg Leu
                85                  90                  95

Ile Gly Ser Ser Ser Ala Thr Cys Ile Ile Ser Gly Asp Thr Val Ile
            100                 105                 110

Trp Asp Asn Glu Thr Pro Ile Cys Asp Arg Ile Pro Cys Gly Leu Pro
        115                 120                 125

Pro Thr Ile Thr Asn Gly Asp Phe Ile Ser Thr Asn Arg Glu Asn Phe
    130                 135                 140

His Tyr Gly Ser Val Val Thr Tyr Arg Cys Asn Pro Gly Ser Gly Gly
145                 150                 155                 160

Arg Lys Val Phe Glu Leu Val Gly Glu Pro Ser Ile Tyr Cys Thr Ser
                165                 170                 175

Asn Asp Asp Gln Val Gly Ile Trp Ser Gly Pro Ala Pro Gln Cys Ile
            180                 185                 190

Ile Pro Asn Lys Cys Thr Pro Pro Asn Val Glu Asn Gly Ile Leu Val
        195                 200                 205

Ser Asp Asn Arg Ser Leu Phe Ser Leu Asn Glu Val Val Glu Phe Arg
    210                 215                 220

Cys Gln Pro Gly Phe Val Met Lys Gly Pro Arg Arg Val Lys Cys Gln
225                 230                 235                 240

Ala Leu Asn Lys Trp Glu Pro Glu Leu Pro Ser Cys Ser Arg Val Cys
                245                 250                 255

Gln Pro Pro Pro Asp Val Leu His Ala Glu Arg Thr Gln Arg Asp Lys
            260                 265                 270

Asp Asn Phe Ser Pro Gly Gln Glu Val Phe Tyr Ser Cys Glu Pro Gly
        275                 280                 285

Tyr Asp Leu Arg Gly Ala Ala Ser Met Arg Cys Thr Pro Gln Gly Asp
    290                 295                 300

Trp Ser Pro Ala Ala Pro Thr Cys Glu Val Lys Ser Cys Asp Asp Phe
305                 310                 315                 320

Met Gly Gln Leu Leu Asn Gly Arg Val Leu Phe Pro Val Asn Leu Gln
                325                 330                 335

Leu Gly Ala Lys Val Asp Phe Val Cys Asp Glu Gly Phe Gln Leu Lys
            340                 345                 350

Gly Ser Ser Ala Ser Tyr Cys Val Leu Ala Gly Met Glu Ser Leu Trp
        355                 360                 365

Asn Ser Ser Val Pro Val Cys Glu Gln Ile Phe Cys Pro Ser Pro Pro
    370                 375                 380

Val Ile Pro Asn Gly Arg His Thr Gly Lys Pro Leu Glu Val Phe Pro
385                 390                 395                 400

Phe Gly Lys Ala Val Asn Tyr Thr Cys Asp Pro His Pro Asp Arg Gly
                405                 410                 415

Thr Ser Phe Asp Leu Ile Gly Glu Ser Thr Ile Arg Cys Thr Ser Asp
            420                 425                 430

Pro Gln Gly Asn Gly Val Trp Ser Ser Pro Ala Pro Arg Cys Gly Ile
        435                 440                 445

Leu Gly His Cys Gln Ala Pro Asp His Phe Leu Phe Ala Lys Leu Lys
    450                 455                 460

Thr Gln Thr Asn Ala Ser Asp Phe Pro Ile Gly Thr Ser Leu Lys Tyr
465                 470                 475                 480

Glu Cys Arg Pro Glu Tyr Tyr Gly Arg Pro Phe Ser Ile Thr Cys Leu
                485                 490                 495

Asp Asn Leu Val Trp Ser Ser Pro Lys Asp Val Cys Lys Arg Lys Ser
            500                 505                 510

Cys Lys Thr Pro Pro Asp Pro Val Asn Gly Met Val His Val Ile Thr
        515                 520                 525

Asp Ile Gln Val Gly Ser Arg Ile Asn Tyr Ser Cys Thr Thr Gly His
    530                 535                 540

Arg Leu Ile Gly His Ser Ser Ala Glu Cys Ile Leu Ser Gly Asn Ala
545                 550                 555                 560

Ala His Trp Ser Thr Lys Pro Pro Ile Cys Gln Arg Ile Pro Cys Gly
                565                 570                 575

Leu Pro Pro Thr Ile Ala Asn Gly Asp Phe Ile Ser Thr Asn Arg Glu
            580                 585                 590

Asn Phe His Tyr Gly Ser Val Val Thr Tyr Arg Cys Asn Pro Gly Ser
        595                 600                 605

Gly Gly Arg Lys Val Phe Glu Leu Val Gly Glu Pro Ser Ile Tyr Cys
    610                 615                 620

Thr Ser Asn Asp Asp Gln Val Gly Ile Trp Ser Gly Pro Ala Pro Gln
625                 630                 635                 640

Cys Ile Ile Pro Asn Lys Cys Thr Pro Pro Asn Val Glu Asn Gly Ile
                645                 650                 655

Leu Val Ser Asp Asn Arg Ser Leu Phe Ser Leu Asn Glu Val Val Glu
            660                 665                 670

Phe Arg Cys Gln Pro Gly Phe Val Met Lys Gly Pro Arg Arg Val Lys
        675                 680                 685

Cys Gln Ala Leu Asn Lys Trp Glu Pro Glu Leu Pro Ser Cys Ser Arg
    690                 695                 700

Val Cys Gln Pro Pro Pro Asp Val Leu His Ala Glu Arg Thr Gln Arg
705                 710                 715                 720

Asp Lys Asp Asn Phe Ser Pro Gly Gln Glu Val Phe Tyr Ser Cys Glu
                725                 730                 735

Pro Gly Tyr Asp Leu Arg Gly Ala Ala Ser Met Arg Cys Thr Pro Gln
            740                 745                 750

Gly Asp Trp Ser Pro Ala Ala Pro Thr Cys Glu Val Lys Ser Cys Asp
        755                 760                 765

Asp Phe Met Gly Gln Leu Leu Asn Gly Arg Val Leu Phe Pro Val Asn
    770                 775                 780

Leu Gln Leu Gly Ala Lys Val Asp Phe Val Cys Asp Glu Gly Phe Gln
785                 790                 795                 800

Leu Lys Gly Ser Ser Ala Ser Tyr Cys Val Leu Ala Gly Met Glu Ser
                805                 810                 815

Leu Trp Asn Ser Ser Val Pro Val Cys Glu Gln Ile Phe Cys Pro Ser
            820                 825                 830

Pro Pro Val Ile Pro Asn Gly Arg His Thr Gly Lys Pro Leu Glu Val
        835                 840                 845

Phe Pro Phe Gly Lys Ala Val Asn Tyr Thr Cys Asp Pro His Pro Asp
    850                 855                 860

Arg Gly Thr Ser Phe Asp Leu Ile Gly Glu Ser Thr Ile Arg Cys Thr
865                 870                 875                 880

Ser Asp Pro Gln Gly Asn Gly Val Trp Ser Ser Pro Ala Pro Arg Cys
                885                 890                 895

Gly Ile Leu Gly His Cys Gln Ala Pro Asp His Phe Leu Phe Ala Lys
            900                 905                 910

Leu Lys Thr Gln Thr Asn Ala Ser Asp Phe Pro Ile Gly Thr Ser Leu
        915                 920                 925

Lys Tyr Glu Cys Arg Pro Glu Tyr Tyr Gly Arg Pro Phe Ser Ile Thr
    930                 935                 940

Cys Leu Asp Asn Leu Val Trp Ser Ser Pro Lys Asp Val Cys Lys Arg

945                    950                    955                    960

Lys Ser Cys Lys Thr Pro Pro Asp Pro Val Asn Gly Met Val His Val
                965                    970                    975

Ile Thr Asp Ile Gln Val Gly Ser Arg Ile Asn Tyr Ser Cys Thr Thr
            980                    985                    990

Gly His Arg Leu Ile Gly His Ser  Ser Ala Glu Cys Ile  Leu Ser Gly
        995                    1000                    1005

Asn Thr  Ala His Trp Ser Thr  Lys Pro Pro Ile Cys  Gln Arg Ile
    1010                    1015                    1020

Pro Cys  Gly Leu Pro Pro Thr  Ile Ala Asn Gly Asp  Phe Ile Ser
    1025                    1030                    1035

Thr Asn  Arg Glu Asn Phe His  Tyr Gly Ser Val Val  Thr Tyr Arg
    1040                    1045                    1050

Cys Asn  Leu Gly Ser Arg Gly  Arg Lys Val Phe Glu  Leu Val Gly
    1055                    1060                    1065

Glu Pro  Ser Ile Tyr Cys Thr  Ser Asn Asp Asp Gln  Val Gly Ile
    1070                    1075                    1080

Trp Ser  Gly Pro Ala Pro Gln  Cys Ile Ile Pro Asn  Lys Cys Thr
    1085                    1090                    1095

Pro Pro  Asn Val Glu Asn Gly  Ile Leu Val Ser Asp  Asn Arg Ser
    1100                    1105                    1110

Leu Phe  Ser Leu Asn Glu Val  Val Glu Phe Arg Cys  Gln Pro Gly
    1115                    1120                    1125

Phe Val  Met Lys Gly Pro Arg  Arg Val Lys Cys Gln  Ala Leu Asn
    1130                    1135                    1140

Lys Trp  Glu Pro Glu Leu Pro  Ser Cys Ser Arg Val  Cys Gln Pro
    1145                    1150                    1155

Pro Pro  Glu Ile Leu His Gly  Glu His Thr Pro Ser  His Gln Asp
    1160                    1165                    1170

Asn Phe  Ser Pro Gly Gln Glu  Val Phe Tyr Ser Cys  Glu Pro Gly
    1175                    1180                    1185

Tyr Asp Leu Arg Gly Ala Ala Ser Leu His Cys Thr Pro Gln Gly
    1190                1195                1200

Asp Trp Ser Pro Glu Ala Pro Arg Cys Ala Val Lys Ser Cys Asp
    1205                1210                1215

Asp Phe Leu Gly Gln Leu Pro His Gly Arg Val Leu Phe Pro Leu
    1220                1225                1230

Asn Leu Gln Leu Gly Ala Lys Val Ser Phe Val Cys Asp Glu Gly
    1235                1240                1245

Phe Arg Leu Lys Gly Ser Ser Val Ser His Cys Val Leu Val Gly
    1250                1255                1260

Met Arg Ser Leu Trp Asn Asn Ser Val Pro Val Cys Glu His Ile
    1265                1270                1275

Phe Cys Pro Asn Pro Pro Ala Ile Leu Asn Gly Arg His Thr Gly
    1280                1285                1290

Thr Pro Ser Gly Asp Ile Pro Tyr Gly Lys Glu Ile Ser Tyr Thr
    1295                1300                1305

Cys Asp Pro His Pro Asp Arg Gly Met Thr Phe Asn Leu Ile Gly
    1310                1315                1320

Glu Ser Thr Ile Arg Cys Thr Ser Asp Pro His Gly Asn Gly Val
    1325                1330                1335

Trp Ser Ser Pro Ala Pro Arg Cys Glu Leu Ser Val Arg Ala Gly
    1340                1345                1350

His Cys Lys Thr Pro Glu Gln Phe Pro Phe Ala Ser Pro Thr Ile
    1355                1360                1365

Pro Ile Asn Asp Phe Glu Phe Pro Val Gly Thr Ser Leu Asn Tyr
    1370                1375                1380

Glu Cys Arg Pro Gly Tyr Phe Gly Lys Met Phe Ser Ile Ser Cys
    1385                1390                1395

Leu Glu Asn Leu Val Trp Ser Ser Val Glu Asp Asn Cys Arg Arg
    1400                1405                1410

Lys Ser Cys Gly Pro Pro Pro Glu Pro Phe Asn Gly Met Val His
    1415                1420                1425

Ile Asn Thr Asp Thr Gln Phe Gly Ser Thr Val Asn Tyr Ser Cys
    1430                1435                1440

Asn Glu Gly Phe Arg Leu Ile Gly Ser Pro Ser Thr Thr Cys Leu
    1445                1450                1455

Val Ser Gly Asn Asn Val Thr Trp Asp Lys Lys Ala Pro Ile Cys
    1460                1465                1470

Glu Ile Ile Ser Cys Glu Pro Pro Pro Thr Ile Ser Asn Gly Asp
    1475                1480                1485

Phe Tyr Ser Asn Asn Arg Thr Ser Phe His Asn Gly Thr Val Val
    1490                1495                1500

Thr Tyr Gln Cys His Thr Gly Pro Asp Gly Glu Gln Leu Phe Glu
    1505                1510                1515

Leu Val Gly Glu Arg Ser Ile Tyr Cys Thr Ser Lys Asp Asp Gln
    1520                1525                1530

Val Gly Val Trp Ser Ser Pro Pro Pro Arg Cys Ile Ser Thr Asn
    1535                1540                1545

Lys Cys Thr Ala Pro Glu Val Glu Asn Ala Ile Arg Val Pro Gly
    1550                1555                1560

Asn Arg Ser Phe Phe Ser Leu Thr Glu Ile Ile Arg Phe Arg Cys
    1565                1570                1575

Gln Pro Gly Phe Val Met Val Gly Ser His Thr Val Gln Cys Gln
    1580                1585                1590

Thr Asn Gly Arg Trp Gly Pro Lys Leu Pro His Cys Ser Arg Val
    1595                1600                1605

Cys Gln Pro Pro Pro Glu Ile Leu His Gly Glu His Thr Leu Ser
    1610                1615                1620

His Gln Asp Asn Phe Ser Pro Gly Gln Glu Val Phe Tyr Ser Cys
    1625                1630                1635

Glu Pro Ser Tyr Asp Leu Arg Gly Ala Ala Ser Leu His Cys Thr
    1640                1645                1650

Pro Gln Gly Asp Trp Ser Pro Glu Ala Pro Arg Cys Thr Val Lys
    1655                1660                1665

Ser Cys Asp Asp Phe Leu Gly Gln Leu Pro His Gly Arg Val Leu
    1670                    1675                    1680

Leu Pro Leu Asn Leu Gln Leu Gly Ala Lys Val Ser Phe Val Cys
    1685                    1690                    1695

Asp Glu Gly Phe Arg Leu Lys Gly Arg Ser Ala Ser His Cys Val
    1700                    1705                    1710

Leu Ala Gly Met Lys Ala Leu Trp Asn Ser Ser Val Pro Val Cys
    1715                    1720                    1725

Glu Gln Ile Phe Cys Pro Asn Pro Pro Ala Ile Leu Asn Gly Arg
    1730                    1735                    1740

His Thr Gly Thr Pro Phe Gly Asp Ile Pro Tyr Gly Lys Glu Ile
    1745                    1750                    1755

Ser Tyr Ala Cys Asp Thr His Pro Asp Arg Gly Met Thr Phe Asn
    1760                    1765                    1770

Leu Ile Gly Glu Ser Ser Ile Arg Cys Thr Ser Asp Pro Gln Gly
    1775                    1780                    1785

Asn Gly Val Trp Ser Ser Pro Ala Pro Arg Cys Glu Leu Ser Val
    1790                    1795                    1800

Pro Ala Ala Cys Pro His Pro Pro Lys Ile Gln Asn Gly His Tyr
    1805                    1810                    1815

Ile Gly Gly His Val Ser Leu Tyr Leu Pro Gly Met Thr Ile Ser
    1820                    1825                    1830

Tyr Ile Cys Asp Pro Gly Tyr Leu Leu Val Gly Lys Gly Phe Ile
    1835                    1840                    1845

Phe Cys Thr Asp Gln Gly Ile Trp Ser Gln Leu Asp His Tyr Cys
    1850                    1855                    1860

Lys Glu Val Asn Cys Ser Phe Pro Leu Phe Met Asn Gly Ile Ser
    1865                    1870                    1875

Lys Glu Leu Glu Met Lys Lys Val Tyr His Tyr Gly Asp Tyr Val
    1880                    1885                    1890

Thr Leu Lys Cys Glu Asp Gly Tyr Thr Leu Glu Gly Ser Pro Trp

1895                    1900                    1905

Ser Gln  Cys Gln Ala Asp Asp  Arg Trp Asp Pro Pro  Leu Ala Lys
    1910                    1915                    1920

Cys Thr  Ser Arg Ala His Asp  Ala
    1925                    1930

## Description

### FIELD OF THE INVENTION

The present invention relates to the use of complement inhibitory proteins, in particular soluble complement receptor type I (sCR1), to treat spinal cord injury.

### BACKGROUND OF THE INVENTION

It is estimated that 7800 people in the United States suffer spinal cord injuries (SCI) each year and that approximately 250,000-400,000 currently suffer from some form of spinal cord injury or spinal dysfunction. (National Spinal Cord Injury Association). Currently there is no cure for spinal cord injury.

SCI is a condition characterized by contusion of the neural tissue with resultant decrease or loss of its ability to function properly and transmit nerve impulses. It has been shown that all neural damage after an SCI event does not occur instantaneously. Following the initial injury, presumably as part of the immune response to the injury, a series of degenerative processes which promote tissue damage beyond the original site of injury are initiated. After the initial mechanical disruption of nerves and nerve fibers at the time of injury, hemorrhaging is usually observed within 30 minutes at the area of damage and may expand over the next few hours. Within several hours following the injury, inflammatory cells, e.g., neutrophils and macrophages, infiltrate the area and cause further damage to the nerve tissue, i.e., cell-mediated damage. These post-traumatic events are referred to as "secondary injury".

It would be advantageous to prevent further damage to the spinal cord and surrounding tissue following a spinal cord injury by treatment as soon as possible after the initial trauma to prevent secondary injury effects.

Currently, the conventional treatment for reducing or minimizing the damage resulting from secondary injury is intravenous injection of the glucocorticoid, methylprednisolone (Bracken et al., JAMA, 277(20): 1597-1604 (1997). Unfortunately, prolonged administration of glucocorticoids has adverse systemic side effects, e.g., increased incidence of sepsis and pneumonia, and a limited therapeutic window.

Recently, Stokes et al. reported improved motor function in Lewis rats having a contusion type injury to the spinal cord after injection with liposomes containing dichloromethylene diphosphonate ("Cl₂MBP") (U.S. Pat. No. 5,932,563). In addition, progress has been reported in improving motor function after spinal cord injury by treatment with α-lipoic acid and/or dihydrolipoic acid (DHL) (U.S. Pat. No. 6,432,434), α_{d} monoclonal antibodies (U.S. Pat. No. 6,432,404), and monosialoganglioside (GM₁) (U.S. Pat. No. 6,620,793).

Neurons and oligodendrocytes may be particularly vulnerable to complement-mediated cell death (Gasque et al.,1995, Journal of Immunology, 154(9): 4726-4733; Agoropoulou et al., 1998, Neuroreport, 9(5): 927-932; Wren et al., 1989, Proc. Natl. Acad. Sci. U.S.A., 86: 9025-9029), suggesting that complement activation can exacerbate damage in the injured CNS by contributing to demyelination and neurodegeneration. Additionally, cell receptor internalization of C5a can also induce apoptosis (Farkas et al., 1998, Neuroscience, 86(3): 903-911; Farkas et al., 1998, J. Physiology, 507(3): 679-687).

Constituting about 10% of the globulins in normal serum, the complement system is composed of many different proteins that are important in the immune system's response to foreign antigens. The complement system becomes activated when its primary components are fragmented and the fragments, alone or with other proteins, activate additional complement proteins resulting in a proteolytic cascade. Activation of the complement system leads to increased vascular permeability, chemotaxis of phagocytic cells, activation of inflammatory cells, opsonization of foreign particles, direct killing of cells and, as seen with spinal cord secondary injury, tissue damage. In view of the continuing need for effective treatments for spinal cord injury, new approaches to addressing post-traumatic phenomena are desirable.

### SUMMARY OF THE INVENTION

We have discovered that administration of a complement inhibitory protein, in particular soluble complement receptor type I, or sCR1, after spinal cord injury, is a surprisingly effective method for inhibiting inflammation and ameliorating the secondary injury effects described above which are associated with spinal cord injury. Soluble CR1 treatment is effective in improving motor function following traumatic spinal cord injury, as demonstrated herein *in vivo* utilizing a rat spinal cord injury model.

Accordingly, in its broadest aspects, the present invention relates to a method for treating spinal cord injury in a vertebrate subject comprising administration of a complement inhibitory protein following spinal cord injury. The treatment is effective to ameliorate or inhibit the damaging effects of secondary injury at the trauma site and to improve motor function. The complement inhibitory protein is preferably administered as soon as possible following a spinal cord injury to prevent the damaging effects of complement activation occurring at the site of injury. In preferred embodiments, the complement inhibitory protein is a soluble complement receptor type I (sCR1), i.e., a truncated, non-membrane-bound fragment of complement receptor type I, which fragment retains the complement regulatory properties of the natural or full-length CR1 protein, in particular the ability to inhibit complement activation and/or the ability to bind complement protein C3b or C4b, or (preferably) both C3b and C4b.

Furthermore, the present invention is directed to a method for ameliorating or inhibiting the secondary injury effects following a spinal cord injury in a vertebrate, which effects are associated with complement activation at the site of the injury. Preferably, said treatment comprises administration of a complement inhibitory protein as soon as possible after the initial injury. Preferably, the complement inhibitory protein is a soluble CR1 comprising at least the N-terminal two short consensus repeats (SCRs) of CR1, more preferably the extracellular domain of full-length human CR1, e.g., comprising amino acids 1-1930 of mature human CR1, most preferably the sCR1 polypeptide having the amino acid sequence of SEQ ID NO:3 (TP10; AVANT Immunotherapeutics, Inc., Needham, MA (USA)). Preferably the complement inhibitory protein is administered in a pharmaceutically acceptable carrier.

In yet another aspect, the present invention provides a method for improving locomotor function in a vertebrate that has suffered a spinal cord injury, the method comprising administration of a complement inhibitory protein after the initial trauma, e.g., to reduce the secondary injury effects caused by complement activation and the resulting inflammatory reaction which leads to further damage to the tissue surrounding the spinal cord. In preferred embodiments, the complement inhibitory protein is a soluble CR1 comprising at least the N-terminal two short consensus repeats (SCRs) of CR1, more preferably the extracellular domain of full-length human CR1, e.g., comprising amino acids 1-1930 of mature human CR1, most preferably the sCR1 polypeptide having the amino acid sequence of SEQ ID NO:3 (TP10; AVANT Immunotherapeutics, Inc.). Preferably, said sCR1 is administered in a pharmaceutically acceptable carrier.

In another aspect, the present invention provides a pharmaceutical composition suitable for inhibiting secondary injury following a spinal cord injury, said composition comprising a complement inhibitory molecule suitable for inhibiting complement activation and the secondary tissue damage at the site of injury resulting from post-traumatic complement activation. In preferred embodiments, the complement inhibitory protein is a soluble CR1 comprising at least the N-terminal two short consensus repeats (SCRs) of CR1, more preferably the extracellular domain of full-length human CR1, e.g., comprising amino acids 1-1930 of mature human CR1, most preferably the sCR1 polypeptide having the amino acid sequence of SEQ ID NO:3.

In yet another aspect, the present invention provides a method for preventing neutrophil activation and neutrophil infiltration of tissue as a result of complement activation at the site of spinal cord injury by administration of a complement inhibitory protein. In preferred embodiments, the complement inhibitory protein is a soluble CR1 comprising at least the N-terminal two short consensus repeats (SCRs) of CR1, more preferably the extracellular domain of full-length human CR1, e.g., comprising amino acids 1-1930 of mature human CR1, most preferably the sCR1 polypeptide having the amino acid sequence of SEQ ID NO:3.

In a particularly preferred embodiment, it is envisioned that the present invention will be suitable for treatment of humans suffering a spinal cord injury by systemic administration of sCR1 as soon as possible after the injury occurs.

The method of this invention can be practiced by using any complement inhibitory protein, such as, for example CR1, factor H, C4-binding protein (C4-BP), membrane cofactor protein (MCP), decay accelerating factor (DAF), or fragments thereof that retain complement inhibiting properties. Alternatively, the complement inhibitory protein may be an antibody specific for a complement protein, an activated complement protein, or a fragment of a complement protein, such antibody being useful to inhibit complement activation (e.g., anti-C3, anti-C5b-9, and the like). However, in preferred embodiments of the present invention, the complement inhibitory protein is human CR1, more preferably a soluble CR1 (sCR1), and most preferably the CR1 polypeptide comprising the extracellular domain of mature human CR1 or the soluble CR1 polypeptide having the amino acid sequence of SEQ ID NO:3.

### DETAILED DESCRIPTION

The present invention is directed to compositions and methods for treating spinal cord injury. In particular, the present invention is directed to compositions and methods for inhibiting the secondary effects known to exacerbate the initial injury and known to result, at least in part, from complement activation. In particular, the present invention is directed to a method for treating spinal cord injury comprising administration of a complement inhibitory protein as soon as possible after the initial spinal cord injury to ameliorate or inhibit the secondary injury effects that occur as a result of the initial trauma. After an initial traumatic event that results in a spinal cord injury (SCI), an inflammatory response at the site of injury results in further damage to the surrounding tissue, thereby exacerbating the effects of the injury and prolonging or preventing recovery. According to the present invention, administration of a complement inhibitory protein such as sCR1, as soon as possible after the initial injury, inhibits or diminishes the subsequent complement-mediated inflammatory response which in turn ameliorates the severity and effects of secondary injury. The treatment described herein can improve locomotor function over time in subjects suffering from a traumatic spinal cord injury.

The method of this invention can be practiced by using any complement inhibitory protein which is effective to block complement activation. Such complement inhibitory proteins include, for example, complement receptor type I (CR1), factor H, C4-binding protein (C4-BP), membrane cofactor protein (MCP), decay accelerating factor (DAF), or fragments thereof that retain complement inhibiting properties, such as the ability to inhibit complement activation, to bind C3b, to bind C4b, or to bind both C3b and C4b. Alternatively, the complement inhibitory protein may be an antibody specific for a complement protein, an activated complement protein, or a fragment of a complement protein, such antibody being effective to inhibit complement activation (e.g., anti-C3, anti-C5b-9, and the like; collectively refered to as "complement-inhibiting antibodies"). Preferably, the complement inhibitory protein used in the methods described herein is a soluble (non-membrane-bound) form of human CR1. Suitable soluble CR1 polypeptides and preparations are described in detail, e.g., in U.S. Pat. No. 5,981,481; U.S. Pat. No. 5,456,909; and U.S. Pat. No. 6,193,979. Special mention is made of a soluble CR1 polypeptide having glycosylation modified to exhibit sialyl Lewis X moieties (sCR1-sLe^{x}), as described in U.S. Pat. No. 6,193,979. More preferably, the method of the invention utilizes a polypeptide comprising the extracellular domain of mature human CR1 (for full-length mature human CR1 sequence, see: SEQ ID NO: 2). Most preferably, the method of the invention and pharmaceutical compositions useful for practicing the invention will include a soluble human CR1 protein having the amino acid sequence of SEQ ID NO:3.

As discussed more fully below, it has been demonstrated herein that administration of sCR1 following spinal cord injury in a rat spinal cord injury model reduces the activity and level of neutrophil infiltration of tissue and reduces the level of expression of complement-related proteins at the injury site as well as improves the overall recovery, i.e., locomotor function in the sCR1-treated rats as compared to normal saline-treated controls. We have thus discovered that administration of a complement inhibitory protein following a spinal cord injury in a vertebrate subject reduces the secondary injury effects caused by complement activation after the initial spinal cord injury.

In a specific embodiment, the invention relates to soluble CR1 polypeptides and their use for the treatment of spinal cord injury. As used herein, the terms "soluble CR1 polypeptides" or "soluble CR1" or "sCR1" shall be used to refer to portions of the full-length CR1 protein which, in contrast to the native CR1 proteins, are not expressed on the cell surface as transmembrane proteins. In particular, CR1 polypeptides which substantially lack a transmembrane region, or, preferably, which comprise all or part of the extracellular portion of CR1, are soluble CR1 polypeptides. In a preferred embodiment, the soluble CR1 polypeptides useful in the present invention are secreted by a cell in which they are expressed.

The human C3b/C4b receptor, termed complement receptor type I (CR1) or CD35, is present on the membranes of erythrocytes, monocytes/macrophages, granulocytes, B cells, some T cells, splenic follicular dendritic cells, and glomerular podocytes. (Fearon D.T., 1980, J. Exp. Med., 152: 20, Wilson, J.G., et al., 1983, J. Immunol., 131: 684). CR1 specifically binds C3b, C4b, and iC3b.

CR1 can inhibit the classical and alternative pathway C3/C5 convertases and act as a cofactor for the cleavage of C3b and C4b by factor I, indicating that CR1 also has complement regulatory functions in addition to serving as a receptor. (Fearon, D.T., 1979, Proc. Natl. Acad. Sci. U.S.A., 76: 5867; Iida, K. I. and Nussenzweig, V., 1981, J. Exp. Med., 153: 1138). In the alternative pathway of complement activation, the bimolecular complex C3b-Bb is a C3 enzyme (convertase). CR1 can bind to C3b thereby promoting the dissociation of fragment Bb from the complex. Furthermore, binding of C3b to CR1 renders C3b susceptible to irreversible proteolytic inactivation by factor I, resulting in the production of inactivated derivatives of C3b (namely, iC3b, C3d and C3dg). In the classical pathway of complement activation, the complex C3bC4bC2a is the C5 convertase. CR1 binds to C4b and/or C3b thereby promoting the dissociation of C2a from the complex. The binding renders C4b and/or C3b susceptible to irreversible proteolytic inactivation by factor I.

Several soluble (non-membrane bound) fragments of CR1 have been generated via recombinant DNA procedures by eliminating the transmembrane and cytoplasmic regions from the DNAs being expressed. See, e.g., Fearon et al., Intl. Patent Publ. WO 89/09220, Oct. 5, 1989; Fearon et al., Intl. Patent Publ. WO 91/05047, Apr. 18, 1991). The soluble CR1 fragments are functionally active, i.e., retaining the ability to bind C3b and/or C4b, inhibiting complement activation, and demonstrating factor I cofactor activity, depending upon the native CR1 regions the CR1 fragments contain. Such constructs inhibit *in vitro* the consequences of complement activation such as neutrophil oxidative burst, complement mediated hemolysis, and C3a and C5a production. A soluble construct, sCR1/pBSCR1c, also has demonstrated *in vivo* activity in a reversed passive Arthus reaction (Fearon et al., 1989, 1991, *supra*; Yeh et al., 1991, J. Immunol., 146:250 (1991), suppressed post-ischemic myocardial inflammation and necrosis (Fearon et al., *supra;* Weisman et al., 1990, Science, 249: 146-151) and extended survival rates following transplantation (Pruitt et al., 1991, J. Surg. Res., 50: 350; Pruitt et al., 1991, Transplantation, 52: 868 (1991)).

The complete cDNA coding sequence of the human CR1 protein is shown in SEQ ID NO:1. The amino acid sequence of mature human CR1 is shown in SEQ ID NO:2.

The isolation of the full-length CR1 gene, expression and purification of the full-length protein and active fragments thereof, and demonstration of activity in the full-length protein and fragments derived from the full-length protein, are described in U.S. Pat. No. 5,981,481, which is incorporated herein by reference.

The complement inhibitory proteins such as sCR1 that are useful in the methods of the present invention are advantageously produced in quantity using recombinant DNA technology to express the protein in a host cell, such as bacterial cells, mammalian cells, or even plant cells. For the complement inhibitory proteins contemplated herein, mammalian host cells, such as Chinese Hamster ovary (CHO) or African Green Monkey kidney (COS) cells, are preferred. The isolated gene encoding the desired protein can be inserted into an appropriate cloning vector. A large number of vector-host systems known in the art may be used. Possible vectors include, but are not limited to, plasmids or modified viruses. The vector system must be compatible with the host cell used. Such vectors include, but are not limited to, bacteriophages such as lambda derivatives, or plasmids such as pBR322 or pUC plasmid or CDM8 plasmid (See, B., 1987, Nature, 329: 840-842) or derivatives of those well-known vectors. Recombinant molecules can be introduced into host cells via transformation, transfection, infection, electroporation, etc.

Recombinant cells producing a preferred form of sCR1 are available from the American Type Culture Collection, Rockville, MD (accession no. CRL 10052). The deposited cells are a Chinese Hamster ovary cell line DUX B 11 carrying plasmid pBSCR1c/pTCSgpt clone 35.6, encoding a soluble CR1 having the amino acid sequence of SEQ ID NO:3. Such sCR1 protein in purified form is produced under the product designation TP10 by AVANT Immunotherapeutics, Inc. (Needham, MA).

After expression in a host cell, the soluble CR1 molecules may be isolated and purified by standard methods including chromatography (e.g., ion exchange, affinity, and sizing column chromatography, high pressure liquid chromatography), centrifugation, differential solubility, or by any other standard technique for the purification of proteins. Preferred purification methods are described in U.S. Pat. No. 6,316,604, U.S. Pat. No. 5,252,216, and U.S. Pat. No. 5,840,858.

Soluble CR1 proteins are therapeutically useful in the modulation of complement-mediated diseases, that is, diseases or conditions characterized by inappropriate or undesired complement activation. A soluble CR1 protein or fragment which can bind C3b or C4b, and/or retain the ability to inhibit the alternative or classical C3 or C5 convertases, and/or retain factor I cofactor activity, can be used to inhibit complement activation. In the present invention, we have demonstrated that soluble CR1 can be used to ameliorate or inhibit undesirable complement activity following traumatic spinal cord injury, e.g., post-traumatic inflammatory conditions resulting from complement activation.

In the method of the invention, a complement inhibitory protein, such as soluble CR1, is administered, preferably intravenously, to a vertebrate subject who has suffered spinal cord injury in order to attenuate complement activation, to reduce or inhibit inflammation associated with the spinal cord injury and to reduce or inhibit detrimental secondary injury effects associated with such inflammation.

In a method of treating spinal cord injury according to the invention, a therapeutically active amount of a complement inhibitory protein or preparation thereof is administered to a subject in need of such treatment. The preferred subject is a human. The amount administered should be sufficient to inhibit complement activation or inhibit the secondary effects of acute inflammation that follow spinal cord injury, such as reducing neutrophil infiltration to injured tissues or reducing neutrophil activity. (See, Example 3, *supra.*) The determination of a therapeutically effective dose is within the capability of practitioners in this art, however, as an example, in embodiments of the method described herein utilizing sCR1 for the treatment of spinal cord injury, an effective dose will be in the range of 0.01-100 mg/kg; preferably 0.1-10 mg/kg, most preferably 1-10mg/kg patient body weight. The pharmaceutical composition should be administered as soon as possible after the spinal cord injury, and repeated doses are contemplated in order to maintain an effective amount, e.g., to reduce or inhibit complement activation. For example, as demonstrated in the examples below, and effective regimen of sCR1 administration consisted of a single injection (6 mg/kg) of sCR1 on the day of spinal cord injury, followed with a similar dose by intravenous injection every day thereafter for the course of treatment.

For administration, the sCR1 or other therapeutic protein may be formulated into an appropriate pharmaceutical composition. Such a composition typically contains a therapeutically active amount of the sCR1 or other protein and a pharmaceutically acceptable excipient or carrier such as saline, buffered saline, phosphate buffers, dextrose, or sterile water. Compositions may also comprise specific stabilizing agents such as sugars, including mannose and mannitol.

Various delivery systems are known and can be used for delivery of CR1 and soluble fragments of CR1, e.g., encapsulation in liposomes, microparticles, or microcapsules. Suitable modes of administration include but are not limited to, intradermal, intramuscular, intraperitoneal, intravenous, subcutaneous, intrathecal, or epidural injection, and oral or pulmonary delivery.

In a preferred embodiment, pharmaceutical compositions for use in the present invention may be formulated in accordance with routine procedures as a pharmaceutical composition for intravenous administration to an individual suffering a spinal cord injury. Typically compositions for intravenous administration are solutions in sterile aqueous buffer. Where necessary, the composition may also include a solubilizing agent and a local anesthetic such as lidocaine to ease pain at the site of injection. Generally, the ingredients will be supplied either separately or mixed together in unit dosage form, for example, as a dry lyophilized powder or water free concentrate in a hermetically sealed container such as an ampoule or sachette indicating the quantity of active agent in activity units. Where the composition is to be administered by injection, an ampoule of sterile water for injection or saline may be provided so that the ingredients may be mixed prior to administration.

A pharmaceutical pack comprising one or more containers filled with one or more of the ingredients of the pharmaceutical composition is also contemplated. This is particularly advantageous as it is perceived that administration of the complement inhibitory protein according to the method of the present invention for the treatment of spinal cord injury could be advantageously administered by emergency personnel on site, e.g., at the site of an automobile accident resulting in a spinal cord injury, as it is critical that the complement inhibitory protein be administered to an individual suffering a spinal cord injury as soon as possible after injury, preferably immediately or within hours or minutes after the initial spinal cord injury occurs.

### EXAMPLES

The following examples illustrate the methods of the present invention. They are provided by way of illustration and not for purposes of limitation.

All data were presented as mean value ± standard deviation (*x* ± *s*). Using SPSS 10.0 statistical analysis software, homogeneity tests for variance were first performed, according to different timepoints, between the treatment and control groups, then *t* tests were performed.

### Example 1

To test whether administration of a complement inhibitory protein can ameliorate or inhibit secondary injury following a traumatic event involving the spinal cord and improve locomotor function over time, Sprague-Dawley (SD) rats (n = 80) all between 250g-300g (Experimental Animal Department of China Medical University) were administered a spinal cord injury by a modified Allen's striking method (Black et al., 1988, Neuro. Surg., 22:51-60) using a 2mm diameter concave plastic pad placed over the target site (T₁₀ segment) and a striking force of 50g/cm.

After injury, the 80 rats were divided randomly into 10 separate groups of 8 rats/group. Five of the 10 groups (n = 40) were comprised of rats receiving sCR1 (TP10, AVANT Immunotherapeutics, Inc.) injections, and the remaining 5 groups (n = 40) were comprised of control rats receiving normal saline injections only. Each of the sCR1 groups and each of the normal saline groups was assigned a separate evaluation time point, i.e., 12 hours, 1 day, 3 days, 7 days, and 14 days following the initial spinal cord injury, respectively. All rats receiving injections of sCR1 (n = 40) were administered caudal intravenous injections of 6mg/kg at 1hour after the initial injury, followed by 1 injection/day (6mg/kg) for the duration of the experiment. Control rats (n = 40) received caudal intravenous injections (6mg/kg) of normal saline at 1hour after initial injury followed by 1 injection/day (6mg/kg) for the duration of the experiment.

### Nerve function evaluation

Recovery of locomotor function of the lower extremities over time was tested using a tilt board method. The rats were placed on the same smooth board at 12 hours, 1 day, 3 days, 7 days, and 14 days after injury. The body axis of the rats was aligned with the longitudinal axis of the tilt board. The longitudinal angle of the tilt board was raised in increments of 5° , and locomotor recovery was evaluated as a function of the maximum tilt angle at which the rat could remain in position on the board. Each rat was tested three times and the mean angle recorded. The results of the tilt board experiment are shown in Table 1.

**Table 1 -- Tilt board experiment (mean angle, x ± s)**

| Timepoint Group | Control (receiving saline) | receiving sCR1 | *t* | P |
|---|---|---|---|---|
| 12 hrs | 33.81 ± 2.75 | 34.12 ± 2.34 | 0.21 | >0.05 |
| 1 day | 34.97 ± 2.69 | 37.57 ± 3.41 | 1.46 | >0.05 |
| 3 days | 36.32 ± 3.72 | 40.91 ± 3.66 | 2.15 | <0.05 |
| 7 days | 38.59 ± 3.12 | 43.24 ± 2.81 | 2.71 | <0.01 |
| 14 days | 44.25 ± 3.78 | 51.58 ± 3.62 | 3.43 | <0.01 |

As seen in Table 1, there was no significant difference in the angle at which sCR1-treated and normal saline-treated (control) rats were able to remain on the tilt board at 12 hours and 1 day after injury; however, the locomotor function of sCR1-treated rats at 3 days, 7 days, and 14 days after treatment was significantly better than the normal saline-treated controls. The results in Table 1 demonstrate that administration of sCR1 following spinal cord injury inhibits the nerve damage at the site of injury, resulting in an improvement in recovery of motor function.

### Example 2

### Pathohistological examination

Three rats from each timepoint group were anesthetized at their respective time points (12 hr, 1 day, 3 days, 7 days, and 14 days after injury), and 1 cm of spinal tissue from the injury site was taken, fixed and dehydrated. 12 µm thick serial sections were made using a freezing microtome and prepared for hematoxylin-eosin (HE) staining or C3c, C9 or CD59 immunohistochemical staining. Rabbit anti-rat C3c antibody was purchased from Zymed Laboratories (Invitrogen Corp., Carlsbad, CA). Rabbit anti-rat C9 and rabbit anti-CD59 antibodies were obtained from Prof. Morgan (Wales University, U.K.). HE staining kits were purchased from Sigma-Aldrich Biotechnology Center (SABC; Sigma-Aldrich Corp., St. Louis, MO).

### Results: HE staining

The results of HE staining were observed microscopically (200X). In the normal saline control group rats, at 12 hrs after injury, there was spotty hemorrhaging in the gray matter and neutrophils began to infiltrate the tissue around the injury site. At 1 day after injury in the normal saline group, neurons were swelling and exhibited dissymmetry of the nuclei. At 3 days after injury, the neurons of the normal saline rats became obviously rounded and swollen, and nuclear pyknosis (indicating apoptosis) and nuclear fragmentation could be seen. In addition, there was a significant amount of neutrophil infiltration of the tissue from the injury site and large spotty hemorrhaging in the gray matter. At 7 days after injury, the remnant neurons decreased in number but some neutrophil infiltration still existed in the gray matter. At 14 days after injury, fewer remnant neurons remained, and a lesser amount of neutrophil infiltration of the gray matter was observed. Porosis could be seen.

In both the sCR1 and control groups, the injured spinal cord deteriorated over time, reaching a peak around day 3 after injury and tending to be stabilized by day 14 after injury. However, in the sCR1-treated groups, all the symptoms associated with secondary effects at the site of injury, i.e., neuron cellular swelling, degeneration, and neutrophil infiltration were notably less severe than in the control group rats as described above.

### Results: C3c immunohistochemical staining

The presence of C3c at the site of injury indicates that complement activation is occurring at the site. The results of the C3c immunohistochemical staining demonstrated that C3c expression levels were lower at the site of injury in the sCR1-treated rats than in the normal saline-treated controls.

Three C3c immunohistochemical staining sections were selected from each rat in every group for analysis. Five high power fields were chosen from the anterior horn to the posterior horn of the gray matter. Average gray value (AG) of C3c-positive reactants was detected using a MetaMorph® automatic colored image analyzer (Universal Imaging Corp., Downington, PA). The average gray value is inversely related to C3c immune reaction intensity. The results of the C3c immunohistochemical staining are shown in Table 2.

**Table 2 -- Image analysis results of C3c positive reactants in injury tissue (AG, x ± s)**

| Timepoint Group | Control (receiving saline) | receiving sCR1 | *t* | P |
|---|---|---|---|---|
| 12 hrs | 54.21 ± 3.08 | 59.37 ± 4.85 | 3.06 | <0.01 |
| 1 day | 48.82 ± 4.75 | 56.25 ± 5.97 | 3.27 | <0.01 |
| 3 days | 39.07 ± 2.26 | 43.81 ± 3.64 | 3.71 | <0.01 |
| 7 days | 47.93 ± 3.99 | 55.20 ± 4.17 | 4.23 | <0.01 |
| 14 days | 58.14 ± 4.72 | 66.23 ± 5.08 | 3.91 | <0.01 |

At 12 hrs after injury, in both the sCR1-treated and normal saline-treated rats, scattered C3c positive expression was observed in some neuron cytomembrane and cytoplasm in the spinal cord anterior horn. At 1 day after injury, C3c expression increased in both groups. At 3 days after injury, high levels of C3c-positive expression were apparent in the remnant neuron cytomembrane, neuropilem, and the neuron cytoplasm. At 7 days after injury, C3c expression decreased gradually. At day 14 after injury, some C3c-positive neurons were still present in the gray matter, and low levels of C3c expression were evident in the neuropilem.

In both the sCR1-treated and control groups, C3c-positive expression in spinal cord injury tissue peaked around day 3 after injury, began to decrease thereafter and was stable at 2 weeks after the injury. However, as seen in Table 2, C3c-positive expression levels were significantly less in the sCR1-treated rats at every timepoint, compared with the normal saline-treated rats (P < 0.01).

### Results: C9 and CD59 immunohistochemical staining

Data from C9 and CD59 immunohistochemical staining were obtained and observed by light microscope (200X) using the same techniques as used in detecting C3c expression.

C3 and C9 are important inherent components of the complement system. C3 is at the confluence of the classical and alternative complement activation pathways and acts as a hub in the complement activation cascade. The existence of C3c, which is a decomposition product of C3 after its inactivation, indicates that activation of the complement system has proceeded to the C3 level. C9, the last molecule that constitutes the membrane attack complex (MAC), is also the most important complement component for activation of the complement system that attacks and destroys target cells. The anti-C9 antibody employed herein was specific for C9 deposited in tissue, which is C9 integrated in the MAC. Therefore, the detection of C9 thoroughly reflected the MAC expression condition.

As with C3c, it was found that C9 was present in the spinal injury tissue at each timepoint after injury in both the sCR1 and the saline (control) groups, which indicates that the complement cascade reaction could be aroused and activated to the end phase in acute spinal cord injury.

The presence of C9 detected in the experiment was similar to that observed in the C3c assays described above. Image analysis results of the C9 detection assays are presented in Table 3.

**Table 3 -- Image analysis results of C9 positive reactants in injury tissue (AG, x ± s)**

| Timepoint Group | Control (receiving saline) | receiving sCR1 | P |
|---|---|---|---|
| 12 hrs | 82.18 ± 4.19 | 88.52 ± 5.03 | <0.01 |
| 1 day | 73.46 ± 3.33 | 81.92 ± 2.24 | <0.01 |
| 3 days | 61.75 ± 2.39 | 71.32 ± 2.32 | <0.01 |
| 7 days | 47.93 ± 5.31 | 81.23 ± 5.30 | <0.01 |
| 14 days | 85.82 ± 5.36 | 95.37 ± 3.06 | <0.01 |

As can be seen from the data of Table 3, the C9 expression in the sCR1-treated groups was significantly less than observed in the saline-treated groups (P < 0.01).

CD59 is a homogeneous restriction factor in the complement end phase. It is an important protective complement regulatory factor, as its main biological activity is to inhibit the continued formation of MAC and to prevent cytolysis.

As with C3c and C9, it was found that CD59 was present in the spinal injury tissue at each timepoint after injury in both the sCR1 and the saline (control) groups, which indicates that the complement cascade reaction could be aroused and activated to the end phase in acute spinal cord injury.

The presence of CD59 detected in the experiment was similar to that observed in the C3c and C9 assays described above. Image analysis results of the CD59 detection assays are presented in Table 4.

**Table 4 -- Image analysis results of C9 positive reactants in injury tissue (AG, x ± s)**

| Timepoint Group | Control (receiving saline) | receiving sCR1 | P |
|---|---|---|---|
| 12 hrs | 95.12 ± 4.89 | 121.31 ± 9.62 | <0.01 |
| 1 day | 87.46 ± 6.34 | 98.54 ± 8.09 | <0.01 |
| 3 days | 69.08 ± 6.18 | 80.01 ± 5.43 | <0.01 |
| 7 days | 80.36 ± 5.01 | 83.57 ± 6.10 | <0.05 |
| 14 days | 96.46 ± 6.97 | 97.35 ± 7.94 | |

As seen in Table 4, the sCR1-treated groups at 12 hours, 1 day, 3 days and 7 days after injury showed a significant difference from the saline-treated controls, indicating that sCR1 was inhibiting complement activation. By day 14, the difference in CD59 expression was no longer significant, which was probably an indication that prolonged treatment with sCR1 was having the protective effect in the spinal injury tissue of reducing destruction of CD59 expressing cells (e.g., neurons, neuroglial cells)

Swelling, degeneration and necrosis of injured neurons were milder at all timepoints in the sCR1-treated groups. Also, less neutrophil infiltration of injured tissue was observed for samples taken from the sCR1-treated groups.

### Example 3

### Myeloperoxidase activity

Myeloperoxidase (MPO) is a marker enzyme for neutrophils, which are the main inflammatory cells involved in acute inflammatory reactions. Myeloperoxidase catalyzes the conversion of hydrogen peroxide (H₂O₂) and chloride anion (Cl⁻) to hypochlorous acid (HOCl). Hypochlorous acid is cytotoxic and is produced by neutrophils to destroy invading bacteria and other pathogens. The presence and levels of myeloperoxidase at the site of injury is thus a direct indication of the activity and level of neutrophil infiltration at the site.

To measure the levels of myeloperoxidase activity at the site of injury, 5 rats from each group at each timepoint, i.e., 12 hours, 1 day, 3 days, 7 days, and 14 days after injury, were anesthesized and a 1cm sample of tissue from the spinal cord injury site was collected. The tissue was preserved in liquid nitrogen and sheared after weighing.

Tribasic potassium phosphate buffer solution with 0.5% cetrimonium bromide (CTBA) was added prior to homogenization. Each sample was then comminuted by ultrasound for 20 minutes at 4°C then centrifuged at 12,500 rpm for 30 minutes. The supernatant was removed and added to 2.9 ml phosphate buffer containing 0.5% dianisidine hydrochloric acid and 0.0005% hydrogen peroxide (pH 7.0). Myeloperoxidase levels were measured spectrophotometrically at 460nm over 2 minutes. One unit of myeloperoxidase activity was defined as the degradation of 1 µmol of hydrogen peroxide per minute at 25°C. Myeloperoxidase levels are expressed as units of myeloperoxidase activity per gram of spinal cord tissue.

The results of the myeloperoxidase assay are shown in Table 5.

**Table 5 -- Myeloperoxidase activity (U/g wet weight, x ± s)**

| Time | NS group | SCR1 group | *t* | p |
|---|---|---|---|---|
| 12 hrs | 0.896 ± 0.062 | 0.543 ± 0.053 | 4.84 | <0.01 |
| 1 day | 1.271 ± 0.065 | 0.932 ± 0.049 | 4.65 | <0.01 |
| 3 days | 1.805 ± 0.073 | 1.327 ± 0.069 | 5.32 | <0.01 |
| 7 days | 1.125 ± 0.047 | 0.764 ± 0.051 | 5.82 | <0.01 |
| 14 days | 0.714 ± 0.054 | 0.401 ± 0.047 | 4.89 | <0.01 |

The levels of myeloperoxidase in the sCR1-treated rats and the normal saline-treated rats began to increase at 12 hours after injury, reached peak levels 3 days after injury, and began to decrease thereafter. However, as seen in Table 5, the myeloperoxidase levels in the sCR1-treated groups were consistently lower at each timepoint than in the control groups (P < 0.01). (See, Table 5.)

These results clearly demonstrate that administration of sCR1 following spinal cord injury reduces the levels and amount of neutrophil infiltration at the site of injury, thereby reducing inflammation and the secondary injury effects associated with inflammation.

The data of the foregoing examples demonstrate that administration of sCR1 following a spinal cord injury in a vertebrate inhibits the activation of complement and inhibits inflammation. Also, improved recovery of locomotor function after the injury was observed following treatment with sCR1. These data demonstrate the suitability of the methods disclosed herein for treating spinal cord injury in vertebrates, including humans.

Although a number of embodiments have been described above, it will be understood by those skilled in the art that modifications and variations of the described compositions and methods may be made without departing from either the disclosure of the invention or the scope of the appended claims. The articles and publications cited herein are incorporated by reference.

## Claims

1. A complement inhibitory protein for use in treating a spinal cord injury in a vertebrate subject.

2. A complement inhibitory protein as claimed in claim 1, wherein said complement inhibitory protein is administered to said vertebrate as soon as possible after said injury.

3. A complement inhibitory protein, as claimed in claim 1 or claim 2, wherein said complement inhibitory protein is selected from complement receptor type I (CR1), factor H, C4-binding protein (C4-BP), membrane cofactor protein (MCP), decay accelerating factor (DAF), fragments thereof that retain complement inhibiting properties, complement-inhibiting antibodies, and sCR1-sLe^{x}.

4. A complement inhibitory protein as claimed in any one of claims 1 to 3, wherein said vertebrate is a human.

5. A complement inhibitory protein as claimed in any one of claims 1 to 4, wherein said complement inhibitory protein is a soluble CR1 protein.

6. A complement inhibitory protein as claimed in claim 5, wherein said soluble CR1 is a polypeptide comprising at least the N-terminal two short consensus repeats of full-length human CR1.

7. A complement inhibitory protein as claimed in claim 6, wherein said soluble CR1 is a polypeptide comprising the extracellular domain of mature human CR1.

8. A complement inhibitory protein as claimed in claim 5, wherein said soluble CR1 has the amino acid sequence of SEQ ID NO:3.

9. A complement inhibitory protein for use in improving the locomotor function of a vertebrate subject suffering from a spinal cord injury.

10. A complement inhibitory protein as claimed in claim 9, wherein said complement inhibitory protein is administered to said subject as soon as possible after said injury.

11. A complement inhibitory protein as claimed in claim 9 or claim 10, wherein said complement inhibitory protein is as defined in claim 3 or any one of claims 5 to 8.

12. A complement inhibitory protein as claimed in anyone of claims 9 to 11, wherein said vertebrate is a human.

13. A soluble complement receptor type I (sCR1) for use in treatment of spinal cord injury.

14. A pharmaceutical composition for use in treating a spinal cord injury comprising a therapeutically effective amount of a soluble CR1 protein and a pharmaceutically acceptable excipient or carrier.

15. A pharmaceutical composition as claimed in claim 14, wherein the soluble CR1 protein is as defined in any one of claims 6 to 8.
